# EUROPÄISCHE PATENTSCHRIFT

(11) **EP 0 354 895 B1**
(45) Veröffentlichungstag und Bekanntmachung des Hinweises auf die Patenterteilung: **27.09.1995**
(21) Anmeldenummer: 89890203.6
(22) Anmeldetag: 31.07.1989
(51) Int. Cl.: A61B 5/14, G01N 1/10, G01N 33/48, G01N 21/17

(54) **Einweg - Messelement**
One-way measuring element
Elément de mesure à sens unique

(30) Priorität: 09.08.1988 AT 2002/88
(43) Veröffentlichungstag der Anmeldung: 14.02.1990
(73) Patentinhaber: AVL Medical Instruments AG, 8207 Schaffhausen (CH)
(72) Erfinder: Leiner, Marco Jean-Pierre, Dr., A-8010 Graz (AT); Harnoncourt, Karl, Dr., A-8010 Graz (AT); Kirchmayer, Gerald, Dipl.-Ing., A-8042 Graz (AT); Kleinhappl, Erich, A-8044 Graz (AT); List, Helmut, Dipl.-Ing., A-8010 Graz (AT); Marsoner, Hermann, Dipl.-Ing. Dr., A-8153 Steinberg (AT); Wolfbeis, Otto S., Dr., A-8046 Graz (AT); Ziegler, Werner E., Dipl.-Ing., A-8043 Graz (AT)
(74) Vertreter: Krause, Walter, Dr. Dipl.-Ing.

(56) Entgegenhaltungen:
- EP-A- 0 121 742
- EP-A- 0 122 420
- EP-A- 0 199 484
- WO-A-85/04719
- US-A- 3 640 267
- US-A- 4 342 964

## Beschreibung

Die Erfindung betrifft ein Einweg-Meßelement, welches zur Analyse von gasförmigen oder flüssigen Proben in ein Analysengerät einsetzbar ist und einen mit einem Kalibriermedium gefüllten Meßkanal eines Sensorteiles mit zumindest einem in einem Meßbereich des Meßkanales angeordneten Sensor aufweist, wobei das ausgangsseitige Ende eines Probenkanals eines Probennahmeteils am Meßkanal anschließbar und die Probe dabei durch das eingangsseitige Ende des Meßkanals unter Verdrängung des Kalibriermediums aus dem Meßbereich in ein mit dem Meßkanal verbundenes Reservoir dem zumindest einen Sensor zuführbar ist, und am anderen reservoirseitigen Ende des Meßkanals ein erster Verschluß vorgesehen ist.

Die US-PS 4 342 964 zeigt eine Vorrichtung der eingangs genannten Art zur elektrochemischen Messung einer Flüssigkeit mit einem in einem Meßkanal angeordneten Meßsensor, sowie einem Kalibriersensor. Die als Elektroden ausgeführten Kalibrier- und Meßsensoren müssen trocken gelagert werden. In einer zusätzlichen Kammer befindet sich während der Lagerung die Kalibrierflüssigkeit, die durch spezielle Einrichtungen erst über die Elektroden geführt werden muß. Durch Druckerhöhung in der Kammer vor der eigentlichen Messung wird das Kalibriermedium dem Kalibrier- und Meßsensor zugeführt. Dabei ist in einer Ausführungsvariante vorgesehen, daß die Probenflüssigkeit über eine dem Meßkanal aufgesetzte Spritze zugeführt wird. In anderen gezeigten Ausführungsvarianten wird eine Kapillare in die Meßeinrichtung eingeführt und auf die Meßelektrode in dem engen Meßkanal aufgesteckt

Ein anderes Meßelement für klinische Analysen ist aus der US-PS 4 654 127 bekanntgeworden. Auf einer Trägerplatte des in ein Analysengerät einsteckbaren Einweg-Meßelementes befindet sich ein als Meßkapillare ausgeführter Meßkanal, welcher in einem Meßbereich mehrere elektrochemische Sensoren aufweist. Dem Eingang des Meßkanals ist ein in zwei Kammern unterteilter Behälter zugeordnet, wobei sich in einer Kammer eine Kalibrierflüssigkeit befindet und in die andere Kammer die zu messende Probe eingefüllt werden kann. Beide Kammern weisen mit durchstechbaren Membranen verschlossene Öffnungen auf, welche mit dem Eingang des Meßkanals in Kontakt gebracht werden können.

Nach dem Einbringen der Probe in die Probenkammer wird - ausgehend von einer Startposition, bei welcher keine der Kammern mit dem Meßkanal in Verbindung steht - der Behälter, beispielsweise durch eine Drehbewegung, in eine Kalibierposition gebracht, die den Kalibrierbehälter verschließende Membran durchstoßen und die Kalibrierflüssigkeit durch die im Meßkanal wirkenden Kapillarkräfte in den Meßbereich gesaugt und analysiert. Danach wird durch eine weitere Drehung des Behälters die Probenkammer an den Eingang des Meßkanals herangeführt und nach dem Durchstoßen der Membran die Probe in den Meßkanal eingebracht und ebenfalls analysiert. Aus den erhaltenen Werten der Proben- und Kalibrierflüssigkeit wird auf die zu messenden Größen in der Probe geschlossen.

Die EP-A 121 742 zeigt ein Gerät zur elektrochemischen Analyse elektrolytischer Bestandteile in einer Probenflüssigkeit, insbesondere einer Körperflüssigkeit mit einer für eine zu analysierende Ionenart selektiven Meßelektrode und einer Referenzhalbzelle, wobei zum Messen der Spannung zwischen Meßelektrode und Referenzhalbzelle eine Spannungsmeßeinrichtung vorgesehen ist. Dabei ist eine Einmal-Meßzelle vorgesehen, welche in ein Kunststoffgehäuse des Analysegerätes einsetzbar ist. Die Einmal-Meßzelle ist bei Messungsbeginn mit einer Referenzflüssigkeit gefüllt. Diese Referenzflüssigkeit muß beim gezeigten Analysegerät ständig in elektrischen Kontakt mit der Referenzhalbzelle stehen und darf bei der eigentlichen Messung der Probe nur in einem geringen Ausmaß aus dem Meßkanal verdrängt werden. Die Einströmöffnung des Sensorteiles muß vor der Messung in eine Probenflüssigkeit eingetaucht werden.

Die gleiche Elektrodeneinheit zur elektrochemischen Analyse elektrischer Bestandteile einer Flüssigkeit ist auch aus der EP-A 0 122 420 bekannt.

Nachteilig bei allen bekannten Ausführungen ist insbesondere deren komplizierter Aufbau und die damit verbundene komplizierte Handhabung. Insbesondere bei der Blutgasanalyse ist es von Nachteil, wenn Blut erst von einem Probennahmeelement, wie beispielsweise einer Kolbenspritze oder einer Kapillare in die Probenkammer gefüllt werden muß, und von dieser erst nach einer weiteren Manipulation in den Meßkanal gelangt, da durch den möglichen Luftzutritt bei dieser Vorgangsweise eine Verfälschung der Blutgaswerte auftritt und reproduzierbare Messungen nur sehr schwer möglich sind. Diese Manipulationen bergen in sich auch die Gefahr, daß das Bedienungspersonal durch Blutproben infiziert wird, was angesichts verschiedener, durch Blut übertragener viraler Erkrankungen als bedeutender Nachteil angesehen werden muß.

Aufgabe der Erfindung ist es, diese Nachteile zu vermeiden und insbesondere ein Einweg-Meßelement vorzuschlagen, welches einen auch für die Massenfertigung einfachen Aufbau aufweist, und bei der Probennahme und Messung einen minimalen Manipulationsaufwand garantiert, wobei der Probentransport von der Entnahme bis in den Meßbereich des Meßkanals möglichst kurz und direkt erfolgen soll.

Diese Aufgabe wird erfindungsgemäß dadurch gelöst, daß der Meßkanal bereits lagermäßig mit dem ein schützendes Lagermedium für die Sensoren bildenden Kalibriermedium vorgefüllt ist, wobei am Sensorteil im Bereich des eingangsseitigen Endes des Meßkanals eine mechanische Kupplung zur direkten Ankoppelung des ausgangsseitigen Endes des Probenkanals an das eingangsseitige Ende des Meßkanals sowie ein bei der Ankoppelung durch das ausgangsseitige Ende des Probenkanals durchtrennbarer, durch eine erste gasdichte Membran gebildeter zweiter Verschluß angeordnet ist, so daß nach Ankoppelung die Probe von dem Probenkanal in den Meßkanal einströmt, und der Probennahmeteil am eingangsseitigen Ende des Probenkanals einen Anschluß für eine Hohlnadel aufweist. Da der Meßkanal des Sensorteiles bereits während der Lagerung mit dem Kalibriermedium gefüllt ist, können allfällige Behälter für Kalibriermedien bzw. deren Mittel zur Zuführung zum Meßkanal vollständig entfallen. Zusätzlich sind die empfindlichen Sensoren während der Bereithaltung und Lagerung des Einweg- bzw. Wegwerfmeßelementes durch das Lagermedium optimal geschützt. Da der Meßkanal an beiden Enden verschlossen ist, kann anstelle einer Kalibrierflüssigkeit auch ein Kalibriergas verwendet werden.

Erfindungsgemäß kann beispielsweise das Kalibrier- und Lagermedium ein unter Atmosphärendruck stehendes, wasserdampfgesättigtes Gasgemisch sein, welches 60 bis 160, vorzugsweise 90 mm Hg O₂ und 20 bis 60, vorzugsweise 35 mm Hg CO₂ sowie ein Inertgas, vorzugsweise Stickstoff, enthält.

Vorteile dabei, insbesondere bei der Verwendung von optischen Sensoren zur Messung des pH-Wertes, des CO₂-Gehaltes und des O₂-Gehaltes von Blut, sind dadurch gegeben, daß durch Variation des Gesamtdruckes jeweils zwei Partialdrücke eingestellt werden können, wodurch eine Zwei-Punkt-Kalibration möglich wird, daß bei angegebener Zusammensetzung eine Kalibration mit Werten erfolgt, welche den physiologischen Normal- bzw. Erwartungswerten sehr nahe kommt, sodaß die Messung in diesem Bereich sehr genau durchgeführt werden kann, sowie daß die Vermischung mit Blut sehr gering gehalten werden kann, und ein gasförmiges Kalibriermedium durch die Blutprobe besonders leicht verdrängt wird.

Für Anwendungsgebiete, wo ein höherer Kalibrieraufwand in Kauf genommen werden kann, ist es erfindungsgemäß auch möglich, daß das Kalibrier- und Lagermedium wasserdampfgesättigte Luft mit den unter Normalbedingungen herrschenden O₂- und CO₂-Partialdrücken ist. Vorteilhafterweise braucht in diesem Fall die Verpackung des Einweg-Meßelementes nicht gasdicht sondern lediglich wasserdampfundurchlässig sein.

Weiters ist beim erfindungsgemäßen Meßelement kein Probenbehälter notwendig, da die Probe aus einem Probennahmeteil direkt in den Meßkanal des Sensorteiles gelangt, wodurch eine Verfälschung der Probenparameter durch Luftzutritt vermieden werden kann, und auch jedwede Kontamination des Bedienungspersonals entfällt. Die miteinander in Kontakt zu bringenden Öffnungen bzw. Kupplungen zum Transport der Kalibrier- und Probenmedien sind auf ein Minimum reduziert, lediglich der Probennahmeteil muß an den Sensorteil angekoppelt werden.

Eine Ausgestaltung der Erfindung sieht vor, daß das Reservoir zur Aufnahme des Kalibrier- und Lagermediums bzw. der Probe ausgangsseitig an den Meßkanal angeschlossen ist. Die in den Meßkanal eingebrachte Probenmenge verdrängt das dort befindliche Kalibriermedium, welches anschließend samt allfälligen Probenüberschüssen in ein Reservoir am Ausgang des Probenkanals gelangt.

In einer Weiterbildung der Erfindung kann der Sensorteil am Ausgang des Meßkanals mit einer zweiten gasdichten, durchstechbaren Membran verschlossen sein, als Reservoir für das Kalibrier- und Lagermedium bzw. für die Probe zwischen Meßkanal und zweiter Membran eine Auffangkammer vorgesehen sein, sowie der Sensorteil nach dem Durchstechen der zweiten Membran an eine Saugeinrichtung des Analysengerätes anschließbar sein. In dieser Ausführungsvariante befindet sich innerhalb des Sensorteils eine an den Meßkanal anschließende Auffangkammer. Über einen von der Auffangkammer wegführenden Kanal, welcher vorerst mit einer durchstechbaren Membran verschlossen ist, wird der Sensorteil an die Saugeinrichtung des Analysengerätes angeschlossen. Dem von der Auffangkammer wegführenden Kanal kann eine optische Füllstandsmeßeinrichtung zugeordnet sein, welche ein Austreten von Kalibrier- oder Probenflüssigkeit in das Analysengerät verhindert.

In einer Variante dieser Ausgestaltung ist es auch möglich, daß in der Auffangkammer des Sensorteiles eine das Kalibrier- und Lagermedium bzw. die Probe am Austritt aus dem Meßelement hindernde, elastische Membran angeordnet ist. Die zweite durchstechbare Membran und allfällige Füllstandsmeßeinrichtungen können dadurch entfallen. Das Kalibrier- und Lagermedium wird bei dieser Ausführungsvariante während der Lagerung durch die eingangsseitig angebrachte durchstechbare Membran und ausgangsseitig durch die in der Auffangkammer ange ordnete elastische Membran im Meßelement eingeschlossen.

Eine andere Weiterbildung der Erfindung sieht vor, daß das Reservoir als Ballon ausgebildet ist, welcher mit einer Quetschmechanik im Analysengerät zusammenwirkt, wobei eine Formänderung des Ballons eine Volumsvergrößerung des Reservoirs nach sich zieht. Vorteilhafterweise werden hier Teile der Saugvorrichtung und das Reservoir für das Kalibriermedium durch einen einzigen Bauteil realisiert. Im Analysengerät befindet sich lediglich eine Mechanik zur Entfaltung des im Ruhezustand abgeflachten Ballons.

In einer besonders vorteilhaften Ausführung der Erfindung bilden der Sensorteil und der Probennahmeteil eine integrierte Einheit, wobei der Probenkanal des Probennahmeteils in einer Kalibrierstellung vor der ersten durchstechbaren Membran des Sensorteils endet und in einer Meßstellung, nach dem Durchstechen der ersten Membran in die zum Meßkanal führende Kupplung einrastet. Damit bildet das gesamte mit der Probe in Berührung kommende Meßelement eine Einheit, welche eine integrierte Probennahme und Messung ermöglicht. Bei der integrierten Probennahme und Messung nach der Erfindung ist der Probennahmeteil untrennbar, aber hinsichtlich der Längsachse verschieb- oder verdrehbar mit dem Sensorteil verbunden. Die Ankopplung des Probenkanals des Probennahmeteils an den Meßkanal des Sensorteils kann z.B. direkt im Analysengerät durch mechanisches Zusammendrücken von Sensorteil und Probennahmeteil mit Hilfe einer im Gerät befindlichen Vorrichtung erfolgen.

Folgende Vorteile liegen auf der Hand: Kein manuelles Ankoppeln von Probennahmeteil und Sensorteil, daher minimales Handling, sowie kein Austreten der Probe, vor allem kein Kontakt mit Blutproben.

Beispielsweise kann in einer erfindungsgemäßen Ausgestaltung vorgesehen sein, daß der Probennahmeteil in axialer Verlängerung des von einem Gehäuse gehaltenen Probenkanals eine mit einer abnehmbaren Kappe versehene Hohlnadel aufweist, daß am Gehäuse des Sensorteiles sowie am Gehäuse des Probennahmeteils eine aus Nuten und Eingreifelementen bestehende Halterung ausgebildet ist, welche den Probennahmeteil in der Kalibrierstellung sowie nach dessen axialer Verschiebung in der Meßstellung festhält. Die Hohlnadel ist hier ein integrierter Bestandteil des Einweg-Meßelementes und braucht nach der Probennahme und Messung nicht separat weggeworfen werden. Nach der Probennahme wird lediglich die Schutzkappe über die Nadel gestülpt und die gesamte Einheit in das Analysengerät eingelegt. Der Übertritt der Probe in den Sensorteil erfolgt an dem der Nadel gegenüberliegenden Ende des Probennahmeteils.

Bei integrierter Probennahme und Messung mit im Analysengerät angeordneter Saugvorrichtung sind folgende Schritte durchzuführen:
1. Einweg-Meßelement mit integriertem Probennahme- und Sensorteil aus der Verpackung nehmen,
2. Kappe von der Hohlnadel entfernen,
3. Probe nehmen,
4. Kappe auf die Hohlnadel aufstecken,
5. Einweg-Meßelement in das Analysengerät einlegen,
6. Analysengerät koppelt den Sensorteil automatisch an die Saugeinrichtung des Gerätes an,
7. Kalibrierung der Sensoren,
8. Gerät koppelt automatisch den Probennahmeteil an den Sensorteil,
9. Analysengerät saugt die Probe in den Meßkanal des Sensorteils,
10. Messung,
11. Einweg-Meßelement wird entfernt und weggeworfen.

Bei der in Punkt 7 angeführten Kalibrierung kann bei Vorliegen eines Kalibriergases auch eine Mehrpunktkalibrierung, vorzugsweise eine Zweipunkt-Kalibrierung, vorgenommen werden, wenn das Saugsystem des Analysengerätes einen Barometeranschluß aufweist und die Kalibrierung in Abhängigkeit unterschiedlicher Druckwerte durchgeführt wird.

Erfindungsgemäß sind hier insbesondere zwei Ausführungsvarianten denkbar, dergestalt, daß der Probenkanal aus einer Glaskapillare besteht und das Gehäuse des Probennahmeteils eine Belüftungsöffnung aufweist. bzw. daß der Probenkanal aus einem Glasrohr besteht und das Gehäuse des Probennahmeteils einen elastischen Bereich aufweist, über welchen nach Druckbetätigung der zur Probennahme notwendige Unterdruck im Glasrohr erzielbar ist. Bei der Ausführung mit der Glaskapillare als Probenkanal gelangt die Probe zum Teil aufgrund der herrschenden Kapillarkräfte und zum Teil aufgrund des Probendruckes (Blutdruck) in den Probenkanal. Bei der zweiten Ausführungsvariante wird der elastische Bereich im Gehäuse des Probennahmeteils, beispielsweise mit dem Daumen leicht eingedrückt und der sich nach Loslassen einstellende Unterdruck zum Einsaugen der Probe verwendet.

Für einige Meßsituationen kann es notwendig sein, die Kalibrierung bereits vor oder während der Probennahmen durchzuführen. Um Zeit zu gewinnen, könnte z.B. ein bereits kalibriertes Meßelement im Analysengerät bereitgehalten werden. Da die Möglichkeit, das Meßelement zwischen Kalibrierung und Messung aus dem Analysengerät zur Probennahme zu entfernen, wegen der Verschmutzungsgefahr im Ankopplungsbereich der Lichtleiter bei Verwendung optischer Sensoren nicht in Betracht kommt, oder zumindest nich ratsam ist, wird in einer weiteren Ausgestaltung der Erfindung vorgeschlagen, daß der als Punktionskit ausgeführte, separate Probennahmeteil eine Glaskapillare und ein Kupplungselement zur Aufnahme einer Hohlnadel aufweist, welches Kupplungselement nach der Probennahme und Entfernung der Hohlnadel an die Kupplung des Sensorteiles ankoppelbar ist. Bei der nichtintegrierten Probennahme und Messung sind Sensorteil und Probennahmeteil getrennt, erst nach der Probennahme werden beide Teile im Gerät manuell oder automatisch zusammengefügt. Der Sensorteil muß dabei zuerst in das Analysengerät in eine dafür vorgesehene Kammer gelegt und der Deckel verschlossen werden. Soll die Ankoppelung manuell erfolgen, so muß der Ankoppelungsbereich des Sensorteils aus dieser Kammer herausragen und von außen für den Benutzer zugänglich sein. Erfolgt die Ankoppelung durch das Analysengerät selbst, so wird der Probennahmeteil in eine zweite, vorzugsweise an die erste anschließende Kammer gelegt, und durch eine Vorrichtung im Gerät angekoppelt. Eine Kalibrierung vor, während oder nach der Probennahme ist so gewährleistet.

Typische Verfahrensschritte bei nicht integrierter Probennahme sind somit folgende:
1. Sensorteil aus der Verpackung nehmen und in das Analysengerät einlegen,
2. Analysengerät koppelt den Sensorteil automatisch an die Saugeinrichtung des Gerätes an,
3. Kalibrierung der Sensoren, währenddessen
4. Probennahmeteil aus der Verpackung nehmen
5. Kappe von der Hohlnadel entfernen,
6. Probe nehmen, Nadel abnehmen und wegwerfen,
7. Probennahmeteil in das Analysengerät einlegen,
8. Gerät koppelt automatisch den Probennahmeteil an den Sensorteil (an den Nadelanschluß),
9. Analysengerät saugt die Probe in den Meßkanal des Sensorteils,
10. Messung,
11. Sensorteil und Probennahmeteil entnehmen und wegwerfen.

Es ist natürlich auch möglich, die Ankoppelung manuell außerhalb des Gerätes vorzunehmen. Dabei muß die Membran, welche den Meßkanal verschließt, vom Benutzer durchstoßen werden. Die außerhalb des Gerätes zusammengefügten Teile werden nun in das Gerät gelegt. Die Vorteile der nichtintegrierten Probennahme, nämlich die unabhängig durchfürbare Probennahme und Kalibrierung, entfallen dabei jedoch.

Eine besondere Ausgestaltung der Erfindung bei nicht integriertem Meß- und Probennahmeteil sieht vor, daß der Probennahmeteil am dem Kupplungselement gegenüberliegenden Ende eine mit der Glaskapillare in Verbindung stehende Kolbenspritze aufweist, welche nach der Ankoppelung des Probennahmeteils an den Sensorteil als Druckvorrichtung zur Einbringung der Probe in den Meßkanal dient. Vorteilhafterweise kann bei dieser Ausführungsvariante die Saugeinrichtung im Analysengerät entfallen. Nach dem Einlegen der Einheit - bestehend aus Meßelement und angekoppelten Punktionskit samt Kolbenspritze - in das Analysengerät, befördert dieses die Probe durch Verschieben des Kolbens aus dem Probennahmeteil in den Meßkanal des Sensorteiles. Es ist natürlich auch möglich, den Probennahmeteil nach der Probennahme und Abnahme der Nadel auf einen im Analysengerät in permanenter Meßbereitschaft gehaltenen Sensorteil aufzustecken und die Probe durch Druck auf den Kolben händisch einzubringen.

Um bei Proben, deren zu messende Parameter eine starke Temperaturabhängigkeit zeigen, reproduzierbare Meßwerte zu erhalten, ist vorgesehen, daß die Glaskapillare in einem möglichst großen Bereich nur zu einem Teil ihres Umfanges in ein Trägerelement des Probennahmeteils eingebettet ist und der vorstehende Teil der Glaskapillare mit einer Thermostatisiereinrichtung des Analysengerätes in Kontakt bringbar ist.

Ein im Aufbau sehr einfach gestaltetes weiteres Einweg-Meßelement, bei dem beide Enden des mit einem flüssigen Kalibrier- und Lagermedium gefüllten Meßkanals an der selben Seite des Sensorteiles angeordnet sind, ist dadurch gekennzeichnet, daß der Eingang und der Ausgang des Meßkanals an der dem Probennahmeteil zugewandten Seite des Sensorteiles liegen, daß dem Ausgang des Meßkanals eine weitere mit einer gasdichten, durchstechbaren Membran versehene Kupplung zugeordnet ist, wobei in eine der Kupplungen eine Glaskapillare des Probennahmeteils und in die andere Kupplung ein mit einem Saugmedium gefülltes Glasrohr einführbar ist. Beispielsweise kann der Meßkanal im Sensorteil U-förmig ausgeführt sein, wobei die beiden an einer Außenfläche des Sensorteils liegenden Kupplungen am Eingang und am Ausgang des Meßkanals von einer gemeinsamen, durchstechbaren Membran verschlossen sind. Nach durchgeführter Kalibrierung wird händisch, oder automatisch vom Analysengerät, ein Probennahmeteil in eine Kupplung und ein mit einem Saugmedium, beispielsweise mit Filterwolle, gefülltes Glasrohr in die zweite Kupplung eingeführt. Die Saugwirkung der Filterwolle ermöglicht dann den Probentransport zu den Sensoren im Meßbereich des Meßkanals.

Erfindungsgemäß kann dabei das Kalibrier- und Lagermedium eine mit O₂ und CO₂ äquilibrierte wäßrige Lösung sein.

Zur besseren Handhabung der letztgenannten Ausführung kann die Glaskapillare des Probennahmeteils und eine mit Filterwolle gefüllte Glaskapillare als Saugeinrichtung parallel ausgerichtet an einem Distanz- und Griffstück befestigt sein.

Ähnlich wie bei den weiter oben angeführten Varianten ist auch hier eine integrierte Einheit von Probennahmeteil und Sensorteil denkbar, wenn in einer Ausgestaltung der Erfindung die Glaskapillare des Probennahmeteils und die mit Filterwolle gefüllte Glaskapillare in einem Gehäuse angeordnet sind und am Gehäuse des Sensorteils sowie am Gehäuse des Probennahmeteils eine aus Nuten und Eingreifelementen bestehende Halterung ausgebildet ist, welche die beiden Glaskapillaren in einer Kalibrierstellung vor den gasdichten Membranen und in einer Meßstellung in den beiden Kupplungen zum Meßkanal festhält.

Eine andere Ausführung im Rahmen der Erfindung sieht vor, daß der Meßkanal bereits lagermäßig mit dem ein schützendes Lagermedium für die Sensoren bildenden Kalibriermedium vorgefüllt ist, wobei beide Enden des Meßkanals an der selben Seite des Sensorteiles angeordnet sind, und wobei am Sensorteil im Bereich der an der dem Probennahmeteil zugewandten Seite liegenden Enden des Meßkanals mechanische Kupplungen zur direkten Ankoppelung des Probennahmeteiles an den Sensorteil angeordnet sind, daß die eingangs- und ausgangsseitigen Kupplungen durch konische Fortsätze des Probennahmeteils verschließbar sind, daß der Probennahmeteil einen Probenkanals mit einem Anschluß für eine Hohlnadel, sowie eine Auffangkammer aufweist, sowie daß nach dem Abnehmen des Probennahmeteils vom Sensorteil, dessen Drehung um 180° und Aufsetzen auf den Sensorteil von der Auffangkammer und vom Probenkanal ausgehende Anschlüsse in den Kupplungen des Sensorteils einrasten, so daß nach Ankoppelung die Probe von dem Probenkanal in den Meßkanal einströmt. Bei dieser Ausführungsvariante ist der Probennahmeteil in zwei Stellungen auf den einen U-förmigen Meßkanal aufweisenden Sensorteil aufsetzbar. In einer ersten Stellung sind die Eingangs- und Ausgangskupplungen des Sensorteils von Fortsätzen des Probennahmeteils verschlossen. In einer zweiten Stellung, bei welcher der Probennahmeteil um 180° gedreht und wieder aufgesetzt wird, wird der Eingang des Meßkanals an den Probenkanal und der Ausgang des Meßkanals an eine Auffangkammer im Probennahmeteil gekoppelt. Der Probennahmeteil kann eine integrierte Hohlnadel aufweisen oder mit einem Anschluß für eine vom Benutzer aufzusteckende Nadel ausgerüstet sein.

Schließlich kann bei allen Ausführungsvarianten vorgesehen sein, daß der Meßkanal des Sensorteiles einen in Fließrichtung der Probe nach dem Meßbereich angeordneten Bereich zur Temperatur- und Füllstandsmessung aufweist. Die Füllstandsmessung kann insbesondere bei undurchsichtigen Proben wie Blut mittels einer Lichtschranke erfolgen. Auch bei optisch durchlässigen Proben können optische Detektoren angewandt werden, wenn beispielsweise Unterschiede in der Lichtbrechung ausgenutzt werden. Zur Temperaturmessung ist im einfachsten Fall einem Bereich der Oberfläche des Sensorteils ein Temperaturfühler des Analysengerätes zugeordnet, über dessen Meßwert auf die Temperatur der Probe geschlossen werden kann. Es ist auch möglich, in den Temperaturmeßbereich des Meßelementes Materialien mit gutem Temperaturleitvermögen einzusetzen.

Schließlich wäre es auch möglich, einen optischen Sensor vorzusehen, mit welchem über die Änderung der Fluoreszenzabklingzeit auf die Temperatur geschlossen wird.

Die Erfindung wird im folgenden anhand von Zeichnungen näher erläutert. Es zeigen in schematischer Darstellung:
- Fig. 1: ein Einweg-Meßelement nach der Erfindung mit integriertem Sensor- und Probennahmeteil, eingesetzt in ein Analysengerät,
- Fig. 2a: eine Ausführungsvariante des Meßelementes nach Fig. 1,
- Fig. 2b: eine Saugeinrichtung des Analysengerätes,
- Fig. 3 und 4: Ausführungsvarianten des Einweg-Meßelementes nach Fig. 1,
- Fig. 5a und Fig. 5b: ein Einweg-Meßelement mit separatem Probennahme- und Sensorteil,
- Fig 5c: einen Schnitt entlang der Linie V-V in Fig. 5a,
- Fig. 6a: eine Ausführungsvariante nach Fig. 5a,
- Fig. 6b: einen Schnitt entlang der Linie VI-VI in Fig. 6a,
- Fig. 7a und 7b: ein Einweg-Meßelement mit separatem Probennahme- und Sensorteil,
- Fig. 8: ein Einweg-Meßelement mit integriertem Sensor- und Probennahmeteil, sowie
- Fig. 9: ein weiteres Einweg-Meßelement.

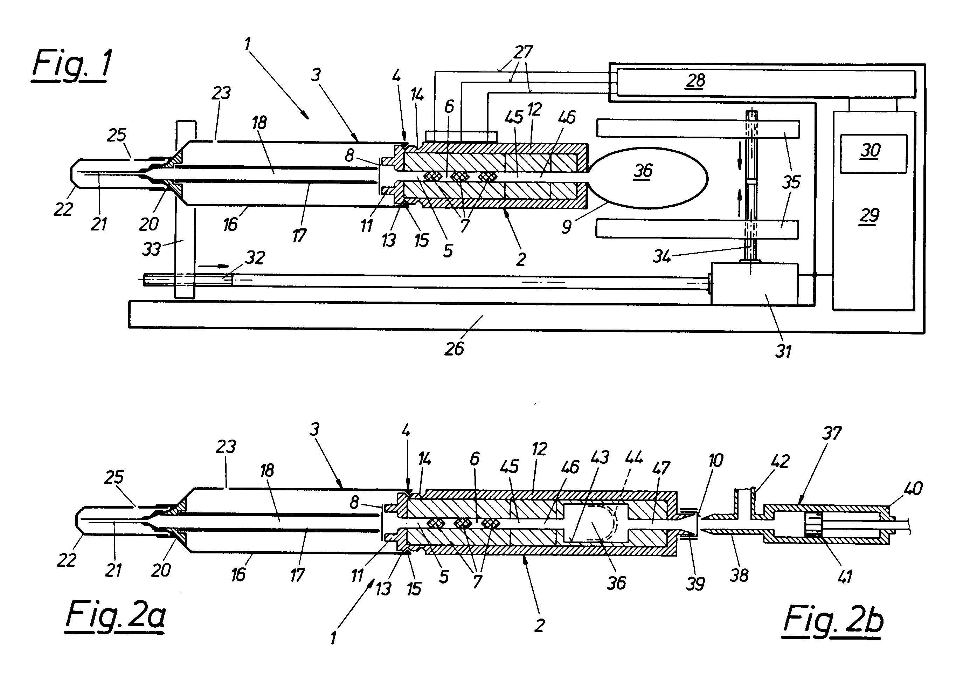

Die in den Fig. 1 bis 4 dargestellten Ausführungsvarianten des Einweg-Meßelementes 1 bestehen jeweils aus einem Sensorteil 2 und einem damit verbundenen Probennahmeteil 3 als integrierte Einheit. Sensorteil 2 und Probennahmeteil 3 werden über eine Halterung 4 verbunden, welche in der dargestellten Kalibrierstellung und in einer Meßstellung einrasten kann.

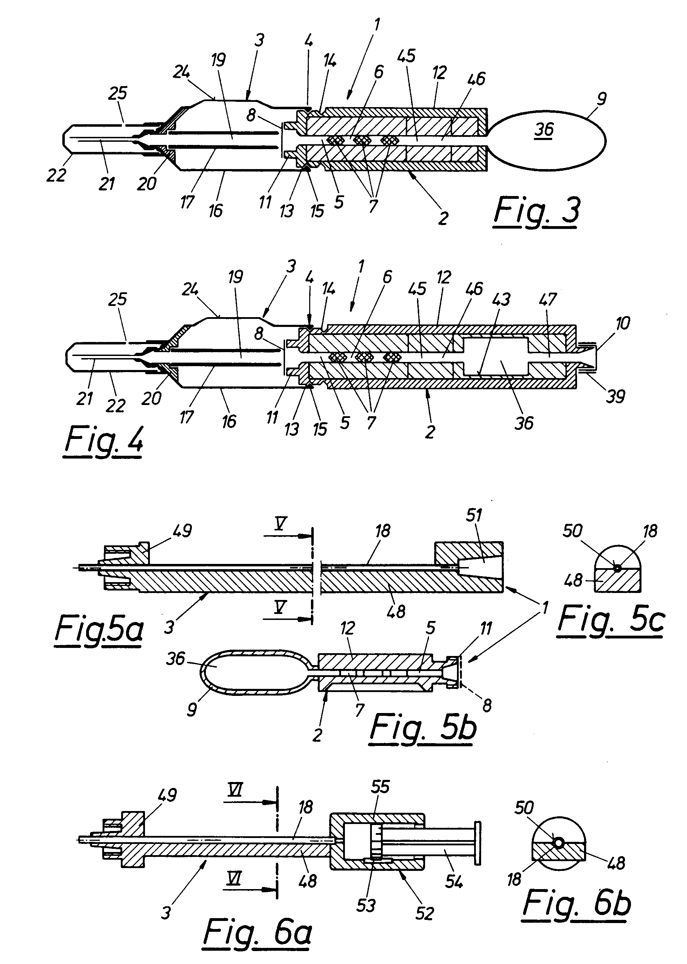

Während der Lagerung des Einweg-Meßelementes, wo dieses einzeln verpackt bereitgehalten wird, befindet sich im Meßkanal 5 mit den im Meßbereich 6 angeordneten Sensoren 7 ein Kalibrier- und Lagermedium, welches durch Verschlüsse 8, 9, 10 am Eingang und am Ausgang des Meßkanals 5 am Austreten aus dem Sensorteil gehindert wird. Der Eingang des Meßkanals 5, welcher eine Kupplung 11 zur direkten Ankoppelung des Probennahmeteils 3 aufweist, ist mittels einer durchstechbaren, gasdichten Membran 8 verschlossen, den Ausgang des Meßkanals verschließt in den Fig. 1 und 3 ein verformbarer elastischer Ballon 9 und in den Fig. 2 und 4 eine weitere durchstechbare, gasdichte Membran 10.

Am Gehäuse 12 des Sensorteiles 2 sind Nuten 13 und 14 angeordnet, welche mit Eingreifelementen 15 am Gehäuse 16 des Probennahmeteils 3 zusammenwirken, wobei in der Kalibrierstellung - welche auch während der Lagerung des Einweg-Meßelementes eingehalten wird - die Eingreifelemente 15 mit der Nut 13 mit der Halterung 4 im Eingriff stehen. In dieser Stellung endet der Probenkanal 17 des Probennahmeteils 3 kurz vor der durchstechbaren Membran 8, koaxial zum Meßkanal 5 und zur Kupplung 11 liegend. Das von der Membran 8 abgewandte Ende des Probenkanals 17, welcher als Glaskapillare 18 (Fig. 1 und 2) oder als Glasrohr 19 (Fig. 3 und 4) ausgeführt werden kann, wird vom Gehäuse 16 des Probennahmeteils 3 gehalten, wobei in diesem Bereich ein Anschluß 20 für eine Hohlnadel 21 vorliegt. Die Hohlnadel 21 wird von einer abnehmbaren Kappe 22 geschützt.

Nach der Entnahme des Einweg-Meßelementes 1 aus der Verpackung wird die Kappe 22 der Hohlnadel 21 abgenommen und eine Probe genommen. Die Probe wird dabei entweder durch die Kapillarkräfte der Glaskapillare 18 entsprechend Fig. 1 oder 2 eingesaugt, wobei das Gehäuse 16 für den Druckausgleich eine Belüftungsöffnung 23 aufweist, oder aktiv vom Benutzer durch Druck auf einen elastischen Bereich 24 des Gehäuses 16 in das Glasrohr 19 transportiert.

Nach der Probennahme wird die Kappe 22, welche ebenfalls eine Belüftungsöffnung 25 aufweist, wieder aufgesetzt und das Meßelement 1 in ein in Fig. 1 schematisch dargestelltes Analysengerät 26 eingelegt, wo die im dargelegten Ausführungsbeispiel optischen Sensoren 7 mit Lichtleitern 27 in Kontakt gebracht werden.

Die über eine nicht weiter dargestellte Anregungs- und Meßeinrichtung 28 des Analysengerätes 26 gewonnenen Meßwerte werden in einer Steuer- und Auswerteeinheit 29 verarbeitet und über eine Display- oder Druckeinheit 30 zur Anzeige gebracht.

Es ist natürlich im Rahmen der Erfindung auch möglich, elektrochemische Sensoren im Meßbereich 6 des Meßkanals 5 vorzusehen, wobei dann lediglich anstelle der optischen Anschlüsse beim Einstecken des Meßelementes 1 in das Analysengerät entsprechende elektrische Anschlüsse vorzusehen wären.

In der Ausführungsvariante nach Fig. 1 werden nun mit Hilfe des sich im Meßkanal befindlichen Kalibriermediums die einzelnen Sensoren kalibriert.

Nach erfolgter Kalibrierung wird der Probennahmeteil 3 automatisch durch das Analysengerät 26 an den Sensorteil 2 angekoppelt. Dabei wird über eine von einem Motor 31 angetriebene Spindel 32 eine Halterung 33 bewegt, welche den Probennahmeteil 3 axial in die Meßstellung verschiebt, in welcher die Eingreifelemente 15 des Gehäuses 16 in der Nut 14 des Sensorteils 2 einrasten. Durch diese Axialverschiebung wird von der die Probe beinhaltenden Glaskapillare 18 des Probennahmeteils 3 die Membran 8 des Sensorteils 2 durchstoßen und die Glaskapillare an die Kupplung 11 des Sensorteils 2 angekoppelt.

Über eine weitere, vom selben Motor 31 angetriebene Spindel 34 wird nun eine Quetschmechanik 25 betrieben, welche eine Formänderung des elastischen Ballons 9 bewirkt, dergestalt, daß sich das Volumen des durch den elastischen Ballon 9 gebildeten Reservoirs 36 vergrößert und die Probe aus dem Probenkanal 17 in den Meßbereich 6 des Meßkanals 5 gesaugt wird. Das Reservoir 36 dient dabei gleichzeitig zur Aufnahme des Kalibriermediums sowie allfälliger Probenüberschüsse. Vorteilhafterweise gelangen bei dieser Ausführung keinerlei Kalibrier- oder Probenmedien in das Analysengerät.

Nach erfolgter Messung wird das Einweg-Meßelement aus dem Analysengerät 26 entnommen und weggeworfen.

Die in Fig. 2a, b dargestellte Ausführung unterscheidet sich von jener nach Fig. 1 vor allem dadurch, daß am Ausgang des Meßkanals 5 eine weitere gasdichte, durchstechbare Membran 10 vorgesehen ist. Nach dem Einlegen dieses Meßelementes in das Analysengerät wird eine in Fig. 2b dargestellte Saugeinrichtung 37 mit einem Saugrohr 38 an eine Kupplung 39 des Sensorteils 2 angekoppelt. Die Saugeinrichtung 37 weist einen in einem Zylinder 40 geführten Kolben 41 auf, dessen Antrieb hier nicht weiter dargestellt ist. Vorteilhafterweise kann hier über einen Barometeranschluß 42 des Saugrohres 38 eine Zweipunkteichung durchgeführt werden, da die Kalibrierung - bei Vorliegen eines Kalibriergases - bei zwei unterschiedlichen Druckwerten durchgeführt werden kann.

Um eine Verschmutzung des Analysengerätes zu vermeiden, ist hier das Reservoir 36 als Auffangkammer 43 zwischen dem Meßkanal 5 und der Membran 10 vorgesehen.

Es ist natürlich auch möglich, in der Auffangkammer eine elastische Membran 44 anzuordnen, wodurch sich ein Austreten von Kalibrier- und Lagermedien verhindern läßt und die durchstechbare Membran 10 hinfällig wäre.

Anschließend an den Meßbereich 6 des Meßkanals 5 sind Bereiche 45, 46 zur Temperatur- und Füllstandsmessung vorgesehen. Bei Ausführungsvarianten nach Fig. 2a, welche keine elastische Membran 44 in der Auffangkammer 43 aufweisen, kann ein weiterer Bereich 47 zur Überlaufkontrolle vorgesehen sein. Für Füllstands- und Überlaufkontrollen eignen sich beispielsweise Lichtschranken; die Temperaturmessung erfolgt im einfachsten Fall an der Außenseite des Gehäuses 12 des Sensorteiles 2.

In allen folgenden Ausführungsvarianten sind gleiche Teile mit gleichen Bezugszeichen versehen. Bei den in den Fig. 5a bis 5c, bzw. 6a und 6b dargestellten Ausführungen sind der als Punktionskit ausgeführte Probennahmeteil 3 und der Sensorteil 2 getrennt verpackt und werden erst nach der Probennahme zusammengekoppelt. Die Ansprüche sind nur auf die Kombination von Probennahmeteil und Sensorteil gerichtet.

Der Probennahmeteil 3 weist eine in ein Trägerelement 48 eingebettete Glaskapillare 18 als Probenkanal auf, welche an ein Kupplungselement 49 zur Aufnahme einer hier nicht dargestellten Hohlnadel anschließt. Nach erfolgter Probennahme und Entfernung der Hohlnadel wird der Probennahmeteil 3 über das Kupplungselement 49 an die Kupplung 11 des Sensorteils 2 angeschlossen und die Membran 8 durchstoßen. Die Glaskapillare ist in einem möglichst großen Bereich ihrer Länge nur zur Hälfte in das Trägerelement 48 eingebettet, wobei der vor allem in den Fig. 5c bzw. 6d sichtbare vorstehende Teil 50 der Glaskapillare nach dem Einsetzen des Meßelementes in das Analysengerät mit einer hier nicht dargestellten Thermostatisiereinrichtung in Kontakt kommt. Am dem Kupplungselement 49 gegenüberliegenden Ende weist der Probennahmeteil 3 ein Sicherheitsvolumen 51 auf, welches bei der Punktion anfallende Probenüberschüsse aufnimmt. Die einzelnen Kupplungen und Kupplungselemente 8, 49 können als Luerverschlüsse ausgeführt sein.

Der in Fig. 6a und 6b dargestellte Probennahmeteil 3 weist am dem Kupplungselement 49 gegenüberliegenden Ende eine mit der Glaskapillare 18 in Verbindung stehende Kolbenspritze 52 auf. Während der Probennahme über die Kapillare erfolgt der dafür notwendige Druckausgleich über eine Nut 53 zwischen Kolben 54 und Zylinder 55 der Kolbenspritze 52. Die Kolbenspritze wird nach dem Ankoppeln des Probennahmeteils 3 an den Sensorteil 2 als Druckvorrichtung zum automatischen oder händischen Einbringen der Probe in den Meßkanal des Sensorteils verwendet.

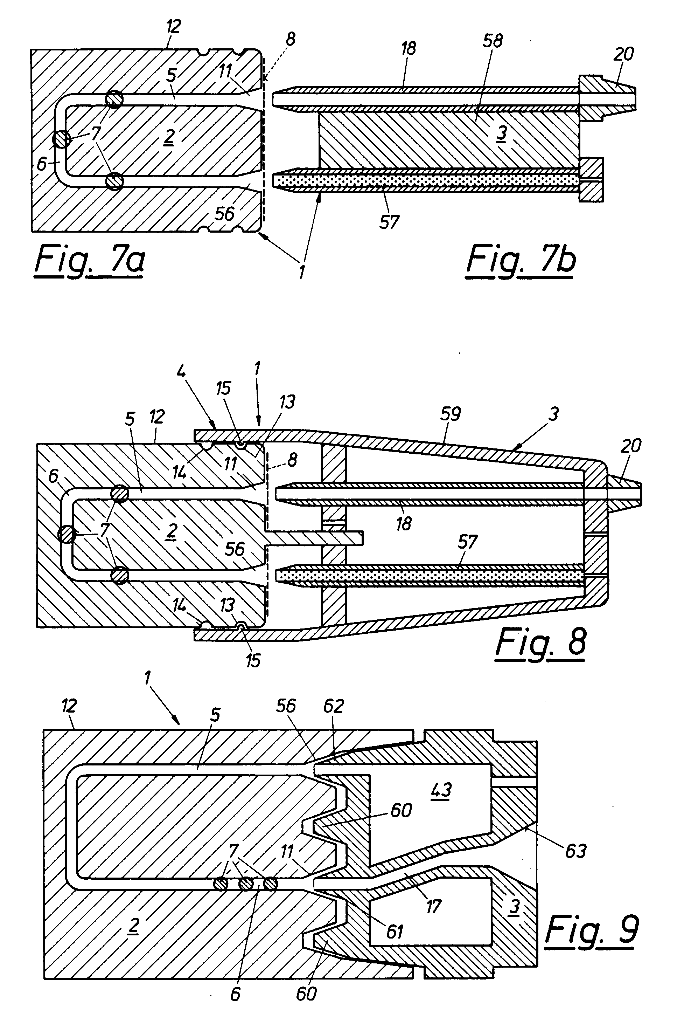

In den Fig. 7 bis 9 weist der Sensorteil 2 jeweils einen U-förmig geführten Meßkanal 5 auf. Sowohl der Eingang als auch der Ausgang des Meßkanals 5 liegen an der dem Probennahmeteil 3 zugewandten Seite des Sensorteils 2.

Bei den Ausführungsvarianten nach Fig. 7 und 8 liegt jeweils neben der Kupplung 11 zur Ankoppelung der Glaskapillare 18 des Probennahmeteils 3 eine weitere Kupplung 56 zur Aufnahme einer mit Filterwolle gefüllten Glaskapillare 57 als Saugeinrichtung für den Probentransport. Vorerst sind beide Kupplungen 11, 56 durch eine gemeinsame durchstechbare Membran 8 verschlossen. Erst nach dem Aufsetzen der durch ein Distanz- und Griffstück 58 (Fig. 7b) verbundenen Glaskapillaren 18 bzw. 57 gelangt die Probe in den Meßbereich 6 des Meßkanals 5 zu den dort angeordneten Sensoren 7. An der dem Sensorteil 2 abgewandten Seite weist der Probennahmeteil 3 einen Anschluß 20 für eine Hohlnadel auf.

Wie aus Fig. 8 ersichtlich, ist auch bei der zuletzt vorgestellten Ausführungsvariante die Ausbildung einer integrierten Einheit aus miteinander verbundenem Meß- und Probennahmeteil denkbar. Dabei sind die Glaskapillare 18 und die mit Filterwolle gefüllte Glaskapillare 57 in einem Gehäuse 59 angeordnet, welches in gleicher Weise wie in den Fig. 1 bis 4 dargestellt, mit dem Gehäuse 12 des Sensorteils 2 über eine Halterung 4 in zwei Stellungen fixierbar ist. In der Kalibrierstellung, wenn die Eingreifelemente 15 in die Nut 13 eingreifen, werden die beiden Glaskapillaren 18 und 57 vor der gasdichten Membran 8 gehalten, in der Meßstellung, wenn die Kapillaren 18 und 57 durch die Membran 8 in die Kupplungen 11 und 56 einrasten, stehen die Eingreifelemente 15 mit der Nut 14 in Verbindung. Da der Probentransport hier von der Saugwirkung der Filterwolle abhängig ist, müssen flüssige Kalibrier- und Lagermedien verwendet werden.

Bei dem in Fig. 9 dargestellten Meßelement 1 werden die eingangs- und ausgangsseitigen Kupplungen 11, 56 des Meßkanals 5 vorerst in der Kalibrierstellung durch konische Fortsätze 60 des Probennahmeteils 3 geschlossen. Nach dem Abnehmen des Probennahmeteils 3 vom Sensorteil 2 und dessen Drehung um 180° kann dieser wieder auf den Sensorteil aufgesetzt werden, wonach - in der Meßstellung - Anschlüsse 61 bzw. 62 ausgehend vom Probenkanal 17 bzw. von einer Auffangkammer 43 in den Kupplungen 11 bzw. 56 des Sensorteiles 2 einrasten. Über bereits beschriebene hier nicht dargestellte Saug- bzw. Pumpvorrichtungen erfolgt dann der Probentransport. Mit 63 ist ein Anschlußkonus für eine Probenentnahmenadel bezeichnet.

## Patentansprüche

1. Einweg-Meßelement, welches zur Analyse von gasförmigen oder flüssigen Proben in ein Analysengerät einsetzbar ist und einen mit einem Kalibriermedium gefüllten Meßkanal (5) eines Sensorteiles (2) mit zumindest einem in einem Meßbereich des Meßkanales (5) angeordneten Sensor aufweist, wobei das ausgangsseitige Ende eines Probenkanals (17) eines Probennahmeteils (3) am Meßkanal (5) anschließbar und die Probe dabei durch das eingangsseitige Ende des Meßkanals (5) unter Verdrängung des Kalibriermediums aus dem Meßbereich in ein mit dem Meßkanal (3) verbundenes Reservoir (36) dem zumindest einen Sensor zuführbar ist, und am anderen reservoirseitigen Ende des Meßkanals (5) ein erster Verschluß (9, 10) vorgesehen ist, **dadurch gekennzeichnet**, daß der Meßkanal (5) bereits lagermäßig mit dem ein schützendes Lagermedium für die Sensoren bildenden Kalibriermedium vorgefüllt ist, wobei am Sensorteil (2) im Bereich des eingangsseitigen Endes des Meßkanals (5) eine mechanische Kupplung (11) zur direkten Ankoppelung des ausgangsseitigen Endes des Probenkanals (17) an das eingangsseitige Ende des Meßkanals (5) sowie ein bei der Ankoppelung durch das ausgangsseitige Ende des Probenkanals (17) durchtrennbarer, durch eine erste gasdichte Membran (8) gebildeter zweiter Verschluß angeordnet ist, so daß nach Ankoppelung die Probe von dem Probenkanal (17) in den Meßkanal (5) einströmt, und der Probennahmeteil (3) am eingangsseitigen Ende des Probenkanals (17) einen Anschluß (20, 49) für eine Hohlnadel (21) aufweist.

2. Einweg-Meßelement nach Anspruch 1, **dadurch gekennzeichnet**, daß das Reservoir (36) zur Aufnahme des Kalibrier- und Lagermediums bzw. der Probe ausgangsseitig an den Meßkanal (5) angeschlossen ist.

3. Einweg-Meßelement nach Anspruch 2 bei der der Verschluß (9, 10) am ausgangsseitigen Ende des Meßkanals (5) durch eine durchstechbare Membran (10) gebildet ist, **dadurch gekennzeichnet**, daß als Reservoir (36) für das Kalibrier- und Lagermedium bzw. für die Probe zwischen Meßkanal (5) und zweiter Membran (10) eine Auffangkammer (43) vorgesehen ist, sowie daß der Sensorteil (2) nach dem Durchstechen der zweiten Membran (10) an eine Saugeinrichtung (37) des Analysengerätes (26) anschließbar ist.

4. Einweg-Meßelement nach Anspruch 2, **dadurch gekennzeichnet**, daß das Reservoir (36) als Ballon (9) ausgebildet ist, welcher mit einer Quetschmechanik (35) im Analysengerät (26) zusammenwirkt, wobei eine Formänderung des Ballons (9) eine Volumenvergrößerung des Reservoirs (36) nach sich zieht.

5. Einweg-Meßelement nach einem der Ansprüche 1 bis 4, **dadurch gekennzeichnet**, daß der Sensorteil (2) und der Probennahmeteil (3) eine integrierte Einheit bilden, wobei der Probenkanal (17) des Probennahmeteils (3) in einer Kalibrierstellung vor der ersten durchstechbaren Membran (8) des Sensorteils (2) endet und in einer Meßstellung, nach dem Durchstechen der ersten Membran (8), in die zum Meßkanal (5) führende Kupplung (11) einrastet.

6. Einweg-Meßelement nach Anspruch 5, **dadurch gekennzeichnet**, daß der Probennahmeteil (3) in axialer Verlängerung des von einem Gehäuse (16) gehaltenen Probenkanals (17) eine mit einer abnehmbaren Kappe (22) versehene Hohlnadel (21) aufweist, daß am Gehäuse (12) des Sensorteiles (2) sowie am Gehäuse (16) des Probennahmeteils (3) eine aus Nuten (13,14) und Eingreifelementen (15) bestehende Halterung (4) ausgebildet ist, welche den Probennahmeteil (3) in der Kalibrierstellung sowie nach dessen axialer Verschiebung in der Meßstellung festhält.

7. Einweg-Meßelement nach Anspruch 6, **dadurch gekennzeichnet**, daß der Probenkanal (17) aus einer Glaskapillare (18) besteht und das Gehäuse (16) des Probennahmeteils (3) eine Belüftungsöffnung (23) aufweist.

8. Einweg-Meßelement nach Anspruch 6, **dadurch gekennzeichnet**, daß der Probenkanal (5) aus einem Glasrohr (19) besteht und das Gehäuse (16) des Probennahmeteils (3) einen elastischen Bereich (24) aufweist, über welchen nach Druckbetätigung der zur Probennahme notwendige Unterdruck im Glasrohr (19) erzielbar ist.

9. Einweg-Meßelement nach Anspruch 3, **dadurch gekennzeichnet**, daß in der Auffangkammer (43) des Sensorteiles (2) eine das Kalibrier- und Lagermedium bzw. die Probe am Austritt aus dem Meßelement (1) hindernde, elastische Membran (44) angeordnet ist.

10. Einweg-Meßelement nach Anspruch 1 bis 4, **dadurch gekennzeichnet**, daß der als Punktionskit ausgeführte Probennahmeteil (3) eine Glaskapillare (18) aufweist, und der Anschluß (49) zur Aufnahme der Hohlnadel als Kupplungselement ausgeführt ist, welches Kupplungselement (49) nach der Probennahme und Entfernung der Hohlnadel an die Kupplung (11) des Sensorteiles (2) ankoppelbar ist.

11. Einweg-Meßelement nach Anspruch 10, **dadurch gekennzeichnet**, daß der Probennahmeteil (3) am dem Kupplungselement (49) gegenüberliegenden Ende eine mit der Glaskapillare (18) in Verbindung stehende Kolbenspritze (52) aufweist, welche nach der Ankoppelung des Probennahmeteils (3) an den Sensorteil (2) als Druckvorrichtung zur Einbringung der Probe in den Meßkanal (5) dient.

12. Einweg-Meßelement nach Anspruch 10 oder 11, **dadurch gekennzeichnet**, daß die Glaskapillare (18) in einem möglichst großen Bereich nur zu einem Teil ihres Umfanges in ein Trägerelement (48) des Probennahmeteils (3) eingebettet ist und der vorstehende Teil der Glaskapillare (18) mit einer Thermostatisiereinrichtung des Analysengerätes in Kontakt bringbar ist.

13. Einweg-Meßelement nach einem der Ansprüche 1 bis 12, **dadurch gekennzeichnet**, daß als Kalibrier- und Lagermedium ein unter Atmosphärendruck stehendes, wasserdampfgesättigtes Gasgemisch vorgesehen ist, welches 60 bis 160, vorzugsweise 90 mm Hg O₂ und 20 bis 60, vorzugsweise 35, mm Hg CO₂ sowie ein Inertgas, vorzugsweise Stickstoff, enthält.

14. Einweg-Meßelement nach einem der Ansprüche 1 bis 12, **dadurch gekennzeichnet**, daß als Kalibrier- und Lagermedium wasserdampfgesättigte Luft mit den unter Normalbedingungen herrschenden O₂- und CO₂-Partialdrücken vorgesehen ist.

15. Einweg-Meßelement nach Anspruch 1, wobei beide Enden des mit einem flüssigen Kalibrier- und Lagermedium gefüllten Meßkanals (5) an der selben Seite des Sensorteiles (2) angeordnet sind, **dadurch gekennzeichnet**, daß der Eingang und der Ausgang des Meßkanals (5) an der dem Probennahmeteil (3) zugewandten Seite des Sensorteiles (2) liegen, daß dem Ausgang des Meßkanals (5) eine weitere mit einer gasdichten, durchstechbaren Membran (8) versehene Kupplung (56) zugeordnet ist, wobei in eine der Kupplungen (11) eine Glaskapillare (18) des Probennahmeteils (3) und in die andere Kupplung (56) ein mit einem Saugmedium gefülltes Glasrohr (57) einführbar ist.

16. Einweg-Meßelement nach Anspruch 15, **dadurch gekennzeichnet**, daß als Kalibrier- und Lagermedium eine mit O₂ und CO₂ äquilibrierte wäßrige Lösung vorgesehen ist.

17. Einweg-Meßelement nach Anspruch 15 oder 16, **dadurch gekennzeichnet**, daß die Glaskapillare (18) des Probennahmeteils (3) und eine mit Filterwolle gefüllte Glaskapillare (57) als Saugeinrichtung parallel ausgerichtet an einem Distanz- und Griffstück (58) befestigt sind.

18. Einweg-Meßelement nach Anspruch 17, **dadurch gekennzeichnet**, daß der Sensorteil (2) und der Probennahmeteil (3) eine integrierte Einheit bilden, wobei die Glaskapillare (18) des Probennahmeteils (3) und die mit Filterwolle gefüllte Glaskapillare (57) in einem Gehäuse (59) angeordnet sind und am Gehäuse (12) des Sensorteils (2) sowie am Gehäuse (59) des Probennahmeteils (3) eine aus Nuten (13,14) und Eingreifelementen (15) bestehende Halterung (4) ausgebildet ist, welche die beiden Glaskapillaren (18,57) in einer Kalibrierstellung vor den gasdichten Membranen (8) und in einer Meßstellung in den beiden Kupplungen (11,56) zum Meßkanal festhält.

19. Einweg-Meßelement, welches zur Analyse von gasförmigen oder flüssigen Proben in ein Analysengerät einsetzbar ist und einen mit einem Kalibriermedium gefüllten Meßkanal (5) eines Sensorteiles (2) mit zumindest einem in einem Meßbereich des Meßkanales (5) eines Sensorteiles angeordneten Sensor aufweist, wobei das ausgangsseitige Ende eines Probenkanals (17) eines Probennahmeteiles (3) am Meßkanal (5) anschließbar und die Probe dabei durch das eingangsseitige Ende des Meßkanals (5) unter Verdrängung des Kalibriermediums in eine Auffangkammer (43) dem zumindest einen Sensor zuführbar ist, und am anderen auffangkammerseitigen Ende des Meßkanals (5) ein Verschluß (9, 10) vorgesehen ist, **dadurch gekennzeichnet**, daß der Meßkanal (5) bereits lagermäßig mit dem ein schützendes Lagermedium für die Sensoren bildenden Kalibriermedium vorgefüllt ist, wobei beide Enden des Meßkanals (5) an der selben Seite des Sensorteiles (2) angeordnet sind, und wobei am Sensorteil (2) im Bereich der an der dem Probennahmeteil (3) zugewandten Seite liegenden Enden des Meßkanals (5) mechanische Kupplungen (11, 56) zur direkten Ankoppelung des Probennahmeteiles (3) an den Sensorteil (2) angeordnet sind, daß die eingangs- und ausgangsseitigen Kupplungen (11, 56) durch konische Fortsätze (60) des Probennahmeteils (3) verschließbar sind, daß der Probennahmeteil (3) einen Probenkanals (17) mit einem Anschluß (63) für eine Hohlnadel, sowie die Auffangkammer (43) aufweist, sowie daß nach dem Abnehmen des Probennahmeteils (3) vom Sensorteil (2), dessen Drehung um 180° und Aufsetzen auf den Sensorteil von der Auffangkammer (43) und vom Probenkanal (17) ausgehende Anschlüsse (61, 62) in den Kupplungen (11, 56) des Sensorteils (2) einrasten, so daß nach Ankoppelung die Probe von dem Probenkanal (17) in den Meßkanal (5) einströmt.

20. Einweg-Meßelement nach einem der Ansprüche 1 bis 19, **dadurch gekennzeichnet**, daß der Meßkanal (5) des Sensorteiles (2) einen in Fließrichtung der Probe nach dem Meßbereich (6) angeordneten Bereich (45,46) zur Temperatur- und Füllstandsmessung aufweist.

21. Einweg-Meßelement nach einem der Ansprüche 1 bis 20, **dadurch gekennzeichnet**, daß im Meßbereich (6) des Meßkanals (5) zumindest ein optischer Sensor (7) angeordnet ist.

22. Einweg-Meßelement nach Anspruch 21, **dadurch gekennzeichnet**, daß zur Analyse von Blutproben im Meßkanal (5) des Sensorteiles (2) optische Sensoren (7) zur Messung des pH-Wertes, des CO₂-Gehaltes sowie des O₂-Gehaltes vorgesehen sind.

## Claims

1. Single-use measuring element capable of use for analysing gaseous or liquid samples in analysing apparatus and comprising a measuring channel (5) of a sensor member (2) filled with a calibrating medium with at least one sensor arranged in a measuring region of the measuring canal (5), the output end of a sample channel (17) of a sample-receiving member (3) being connectable to the measuring channel (5) and the sample thereby being feedable through the input end of the measuring channel (5) under the urging of the calibrating medium from the measuring region into a reservoir (36) connected to the measuring channel (3) to the at least one sensor, and a first closure (9, 10) being provided at the reservoir end of the measuring channel (5), characterised in that the measuring channel (5) is already filled previously for carrier purposes with the calibrating medium forming a protective carrier medium for the sensors, there being arranged on the sensor member (2) in the region of the input end of the measuring channel (5) a mechanical coupling (11) for the direct coupling-in of the output end of the sample channel (17) to the input end of the measuring channel (5) as well as a second closure formed by a first gas-tight membrane (8) piercable on coupling-in by the output end of the sample channel (17), so that after coupling-in the sample flows from the sample channel (17) into the measuring channel (5), and the sample-receiving member (3) has a connection (20, 49) for a hollow needle (21) at the input end of the sample channel (17).

2. Single-use measuring element according to claim 1, characterised in that the reservoir (36) for receiving the calibrating and carrier medium and the sample is connected at its outlet end to the measuring channel (5).

3. Single-use measuring element according to claim 2 in which the closure (9, 10) is formed by a piercable membrane at the outlet end of the measuring channel (5), characterised in that a collecting chamber (43) is provided between the measuring channel (5) and the second membrane (10) as the reservoir (36) for the calibrating and carrier medium and for the sample, and that the sensor member (2) is capable of connection to a suction device (37) of the analysing equipment (26) after the piercing of the second membrane (10).

4. Single-use measuring element according to claim 2, characterised in that the reservoir (36) is in the form of a balloon (9) which co-operates with a pinching mechanism (35) in the analysing equipment (26), whereby a change in shape of the balloon (9) causes an increase in the volume of the reservoir (36).

5. Single-use measuring element according to one of claims 1 to 4 characterised in that the sensor member (2) and the sample-receiving member (3) form an integral unit, the sample channel (17) of the sample-receiving member (3) terminating, in a calibrating position, in front of the first piercable membrane (8) of the sensor member (2) and in a measuring position, after piercing of the first membrane (8), engaging in the coupling (11) leading to the measuring channel (5).

6. Single-use measuring element according to claim 5, characterised in that the sample-receiving member (3) has, in an axial prolongation of the sample channel (17) retained by a housing (16), a hollow needle (21) provided with a removable cap (22), that a retaining means (4) comprising grooves (13, 14) and engaging elements (15) is provided on the housing (12) of the sensor member (2) and on the housing (16) of the sample-receiving member (3), which retaining means secures the sample-receiving member (3) in the calibrating position and, after its axial displacement, in the measuring position.

7. Single-use measuring element according to claim 6, characterised in that the sample channel (17) comprises a glass capillary tube (18) and the housing (16) of the sample-receiving member (3) has a venting opening (23).

8. Single-use measuring element according to claim 6, characterised in that the sample channel (5) comprises a glass tube (19) and the housing (16) of the sample-receiving member (3) has an elastic region (24), via which, after actuation, the negative pressure in the glass tube (19) necessary for drawing in the sample is obtained.

9. Single-use measuring element according to claim 3 characterised in that an elastic membrane (44) which prevents the calibrating and carrier medium and the sample emerging from the measuring element (1) is arranged in the collecting chamber (43) of the sensor member (2).

10. Single-use measuring element according to claim 1 to 4, characterised in that the sample-receiving member (3), constructed as a puncturing kit, comprises a glass capillary tube (18) and the connecting element (49) for receiving the hollow needle, which connecting element (49) can be connected to the coupling (11) of the sensor member (2) after receiving the sample and removal of the hollow needle.

11. Single-use measuring element according to claim 10, characterised in that the sample-receiving member (3) has on that end of it which is opposite the connecting element (49) a piston syringe (52) in communication with the glass capillary tube (18), the syringe serving as pressure means for introducing the sample into the measuring channel (5) after connection of the sample-receiving member (3) to the sensor member (2).

12. Single-use measuring element according to claim 10 or 11 characterised in that the glass capillary tube (18) is embedded in a carrier element (48) of the sample-receiving member (3) in as large as possible a region over only a part of its periphery, and the exposed portion of the glass capillary tube (18) is capable of being brought into contact with a thermostat action device of the analysing equipment.

13. Single-use measuring element according to one of claims 1 to 12, characterised in that a gas mixture at atmospheric pressure and saturated with water vapour is provided as the calibrating and carrier medium, containing 60mm to 160mm, preferably 90mm HgO₂ and 20mm to 60mm preferably 35mm HgCO₂ and an inert gas, preferably nitrogen.

14. Single-use measuring element according to one of claims 1 to 12, characterised in that air saturated with water vapour with the O₂ and CO₂ partial pressures which prevail under normal conditions is provided as the calibrating and carrier medium.

15. Single-use measuring element according to claim 1, in which both ends of the measuring channel (5) filled with a fluid calibrating and carrier medium are connected to the same end of the sensor member (2), characterised in that the input and the outlet of the measuring channel (5) lie at that end of the sensor member (2) which is towards the sample-receiving member (3), that a further coupling (56) provided with a gas-tight piercable membrane (8) is fitted to the outlet of the measuring channel (5), whereby a glass capillary tube (18) of the sample-receiving member (3) is introduced into one of the couplings (11) and a glass tube (57) filled with a suction medium is introduced into the other coupling (56).

16. Single-use measuring element according to claim 15, characterised in that an aqueous solution stabilised with O₂ and CO₂ is provided as the calibrating and carrier medium.

17. Single-use measuring element according to claim 15 or 16, characterised in that the glass capillary tube (18) of the sample-receiving member (3) and a glass capillary tube (57) filled with a wool filter forming the suction medium are secured, directed parallel with one another, on a spacing and securing component (58).

18. Single-use measuring element according to claim 17, characterised in that the sensor member (2) and the sample-receiving member (3) form an integral unit, the glass capillary tube (18) of the sample-receiving member (3) and the glass capillary tube (57) filled with a wool filter being arranged in a housing (59) and a retaining means (4) comprising grooves (13, 14) and engaging elements (15) being formed on the housing (12) of the sensor member (2) as well as on the housing (59) of the sample-receiving member (3), the retaining means securing the two glass capillary tubes (18, 57) to the measuring channel in a calibrating position in front of the gas-tight membrane (8) and, in a measuring position, within the two couplings (11, 56).

19. Single-use measuring element capable of use for analysing gaseous or liquid samples in analysing apparatus and having a measuring channel (5) of a sensor member (2) filled with a calibrating medium with at least one sensor arranged in a measuring region of the measuring channel (5) of a sensor member, the output end of a sample channel (17) of a sample-receiving member (3) being connectable to the measuring channel (5) and the sample thereby being feedable through the input end of the measuring channel (5) under the urging of the calibrating medium in a receiving chamber (43) to the at least one sensor, and a closure (9, 10) being provided on the other receiving chamber end of the measuring channel, characterised in that, the measuring channel (5) is already filled for carrier purposes with the calibrating medium forming a protective carrier medium for the sensors, both ends of the measuring channel (5) being connected to the same end of the sensor member (2), and mechanical couplings (11, 56) for direct coupling of the sample-receiving member (3) to the sensor member (2) being arranged on the sensor member (2) in the region of the ends of the measuring channel (5) lying towards the sample-receiving member (3), that the couplings (11, 56) at the inlet and outlet ends are closable by conical extensions (60) of the sample-receiving member (3), that the sample-receiving member (3) has a sample channel (17) with a connection (63) for a hollow needle as well as the collecting chamber (43), and that after removal of the sample-receiving member (3) from the sensor member (2), its rotation through 180º and placing on the sensor member, connections (61, 62) leading from the collecting chamber (43) and from the sample channel (17) engage in the couplings (11, 56) of the sensor member (2) so that after coupling-on the sample flows from the sample channel (17) into the measuring channel (5).

20. Single-use measuring element according to one of claims 1 to 19, characterised in that the measuring channel (5) of the sensor member (2) has a region (45, 46) for measuring the temperature and the state of filling, arranged following the measuring region (6) in the direction of flow of the sample.

21. Single-use measuring element according to one of claims 1 to 20, characterised in that at least one optical sensor (7) is arranged in the measuring region (6) of the measuring channel (5).

22. Single-use measuring element according to claim 21, characterised in that, for analysing blood samples, optical sensors (7) are provided in the measuring channel (5) of the sensor member (2) for measuring the pH value, the CO₂ content and the O₂ content.

## Revendications

1. Elément de mesure jetable qui se monte dans un appareil d'analyse pour l'analyse d'échantillons gazeux ou liquides et comporte un canal de mesure (5) rempli d'un fluide de calibrage dans une partie de capteur (2) avec au moins un capteur prévu dans la zone de mesure du canal de mesure (5), l'extrémité de sortie d'un canal d'échantillon (17) d'une partie de prélèvement d'échantillon (3) pouvant se raccorder au canal de mesure (5) et l'échantillon étant fourni par l'extrémité d'entrée du canal de mesure (5) tout en refoulant le fluide de calibrage de la zone de mesure dans un réservoir (36) relié au canal de mesure (3), pour être fourni au moins à un capteur et à l'autre extrémité du canal de mesure (5), du côté du réservoir, il est prévu un premier moyen de fermeture (9, 10), caractérisé en ce que le canal de mesure (5) est déjà pré-rempli au stockage d'un fluide de calibrage formant un fluide de conservation protecteur pour les capteurs, la partie de capteur (2) ayant, au niveau de l'extrémité d'entrée du canal de mesure (5), un moyen d'accouplement mécanique (11) pour le raccordement direct de l'extrémité de sortie du canal d'échantillon (17) à l'extrémité d'entrée du canal de mesure (5) ainsi qu'un second moyen de fermeture constitué par une première membrane (8) étanche aux gaz qui est percée par l'extrémité de sortie du canal d'échantillon (17) lors du raccordement, de sorte qu'après raccordement l'échantillon coule du canal d'échantillon (17) dans le canal de mesure (5) et la partie de prélèvement d'échantillon (3) comporte, à l'extrémité d'entrée du canal d'échantillon (17), un moyen de raccordement (20, 49) pour une canule (21).

2. Elément de mesure jetable selon la revendication 1, caractérisé en ce que le réservoir (36) recevant le fluide de calibrage et de conservation ou l'échantillon, est relié en sortie au canal de mesure (5).

3. Elément de mesure jetable selon la revendication 2, dont le moyen de fermeture (9, 10) à l'extrémité de sortie du canal de mesure (5) est constitué par une membrane (10) qui peut être transpercée, caractérisé en ce que le réservoir (36) pour le fluide de calibrage et de conservation ou pour l'échantillon est constitué par une chambre de réception (43) prévue entre le canal de mesure (5) et la seconde membrane (10) et la partie de capteur (2) peut être raccordée à une installation d'aspiration (37) de l'appareil d'analyse (26) après percement de la seconde membrane (10).

4. Elément de mesure jetable selon la revendication 2, caractérisé en ce que le réservoir (36) est en forme de ballon (9) qui coopère avec un mécanisme d'écrasement (35) de l'appareil d'analyse (26), la modification de forme du ballon (9) provoquant une augmentation de volume du réservoir (36).

5. Elément de mesure jetable selon l'une des revendications 1 à 4, caractérisé en ce que la partie de capteur (2) et la partie de prélèvement d'échantillon (3) constituent un ensemble intégré, le canal à échantillon (17) de la partie de prélèvement d'échantillon (3) se terminant dans une position de calibrage, en avant de la première membrane (8) transperçable de la partie de capteur (2) et dans une position de mesure, après percement de la première membrane (8), le canal (17) s'accroche dans le moyen d'accouplement (11) conduisant au canal de mesure (5).

6. Elément de mesure jetable selon la revendication 5, caractérisé en ce que la partie de prélèvement d'échantillon (3) comporte dans le prolongement axial du canal d'échantillon (17) maintenu par le boîtier (16), une canule (21) munie d'un capuchon (22) amovible, et un dispositif de fixation (4) comprenant des rainures (13, 14) et des éléments de prise (15) étant réalisé sur le boîtier (12) de la partie de capteur (2) ainsi que sur le boîtier (16) de la partie de prélèvement d'échantillon (3), ce moyen de fixation bloquant la partie de prélèvement d'échantillon (3) dans la position de calibrage et après son coulissement axial, en position de mesure.

7. Elément de mesure jetable selon la revendication 6, caractérisé en ce que le canal d'échantillon (17) est un tube capillaire en verre (18) et le boîtier (16) de la partie de prélèvement d'échantillon (3) comporte un orifice de prise d'air (23).

8. Elément de mesure jetable selon la revendication 6, caractérisé en ce que le canal d'échantillon (5) est un tube de verre (19) et le boîtier (16) de la partie de prélèvement d'échantillon (3) comporte une zone élastique (24) par laquelle, par mise en oeuvre de la pression, on réalise dans le tube en verre (19), la dépression nécessaire au prélèvement de l'échantillon.

9. Elément de mesure jetable selon la revendication 3, caractérisé par une membrane élastique (44) prévue dans la chambre de réception (43) de la partie de capteur (2), qui interdit au fluide de calibrage et de conservation ou à l'échantillon de sortir de l'élément de mesure (1).

10. Elément de mesure jetable selon les revendications 1 à 4, caractérisé en ce que la partie de prélèvement d'échantillon (3), réalisée sous la forme d'un ensemble de ponction, comporte un tube capillaire en verre (18) et le raccord (49) pour recevoir la canule est réalisé sous la forme d'un élément d'accouplement, cet élément d'accouplement (49) pouvant être raccordé à l'élément d'accouplement (11) de la partie de capteur (2) après prélèvement de l'échantillon et enlèvement de la canule.

11. Elément de mesure jetable selon la revendication 10, caractérisé en ce que la partie de prélèvement d'échantillon (3) comporte, à l'extrémité opposée à l'élément d'accouplement (49), une seringue à piston (52) et qui, après raccordement de la partie de prélèvement d'échantillon (3) sur la partie de capteur (2) constitue le dispositif de pression pour introduire l'échantillon dans le canal de mesure (5).

12. Elément de mesure jetable selon la revendication 10 ou 11, caractérisé en ce que le tube capillaire en verre (18) est noyé dans une zone aussi grande que possible mais ne correspondant qu'à une partie de sa périphérie dans un élément de support (48) de la partie de prélèvement d'échantillon (3) et la partie en saillie du tube capillaire en verre (18) peut être mise en contact avec une installation thermostatique de l'appareil d'analyse.

13. Elément de mesure jetable selon l'une des revendications 1 à 12, caractérisé en ce que le fluide de calibrage et de conservation est un mélange de gaz saturé de vapeur d'eau, à la pression atmosphérique, qui contient de l'oxygène à 60-160 et de préférence à 90 mm Hg en O₂ et entre 20 et 60 et de préférence 35 mm Hg en CO₂ ainsi qu'un gaz inerte, de préférence de l'azote.

14. Elément de mesure jetable selon l'une des revendications 1 à 12, caractérisé en ce que le fluide de calibrage et de stockage est de l'air saturé de vapeur d'eau sous des conditions normales, aux pressions partielles de O₂ et de CO₂.

15. Elément de mesure jetable selon la revendication 1, dont les deux extrémités du canal de mesure (5) rempli d'un liquide de calibrage et de conservation, sont prévues du même côté de la partie de capteur (2), caractérisé en ce que l'entrée et la sortie du canal de mesure (5) sont situées du côté de la partie de capteur (2) tournée vers la partie de prélèvement d'échantillon (3), la sortie du canal de mesure (5) comportant un autre moyen d'accouplement (56) muni d'une membrane (8) étanche aux gaz, susceptible d'être percée, et dans l'un des moyens d'accouplement (11) on peut introduire un tube capillaire en verre (18) de la partie de prélèvement d'échantillon (3) et dans l'autre moyen d'accouplement (56) on peut introduire un tube de verre (57) rempli d'un fluide d'aspiration.

16. Elément de mesure jetable selon la revendication 15, caractérisé en ce que le fluide de calibrage et de conservation est une solution aqueuse équilibrée en O₂ et CO₂.

17. Elément de mesure jetable selon les revendications 15 ou 16, caractérisé en ce que le tube capillaire en verre (18) de la partie de prélèvement d'échantillon (3) et un tube capillaire en verre (57) rempli d'une laine filtrante, sont alignés parallèlement pour constituer un dispositif d'aspiration en étant fixés sur une pièce d'écartement et de préhension (58).

18. Elément de mesure jetable selon la revendication 17, caractérisé en ce que la partie de capteur (2) et la partie de prélèvement d'échantillon (3) forment un ensemble intégré, le tube capillaire en verre (18) de la partie de prélèvement d'échantillon (3) et le tube capillaire en verre (57) rempli d'une laine filtrante sont logés dans un boîtier (59) et le boîtier (12) de la partie de capteur (2) ainsi que le boîtier (59) de la partie de prélèvement d'échantillon (3) comportent un moyen de fixation (4) formé de rainures (13, 14) et d'éléments de prise (15), ce moyen de fixation maintenant réunis les deux tubes capillaires en verre (18, 57) dans une position de calibrage devant les membranes étanches au gaz (8) et dans une position de mesure dans les deux moyens d'accouplement (11, 56) par rapport au canal de mesure.

19. Elément de mesure jetable destiné à un appareil d'analyse pour analyser des échantillons gazeux ou liquides et comportant un canal de mesure (5) rempli d'un fluide de calibrage dans une partie de capteur (2) avec au moins un capteur prévu dans une plage de mesures du canal de mesure (5) de la partie de capteur, l'extrémité de sortie d'un canal d'échantillon (17) d'une partie de prélèvement d'échantillon (3) pouvant se raccorder au canal de mesure (5) pour fournir ainsi l'échantillon par l'extrémité d'entrée du canal de mesure (5) en refoulant le fluide de calibrage dans une chambre de réception (43) d'au moins un capteur et dont l'extrémité du canal de mesure (5), du côté de la chambre de réception, comporte un moyen de fermeture (9, 10), caractérisé en ce que le canal de mesure (5) est déjà pré-rempli au stockage d'un fluide de calibrage constituant un fluide de conservation, protecteur, pour les capteurs, les deux extrémités du canal de mesure (5) se trouvant du même côté de la partie de capteur (2) et au niveau des extrémités du canal de mesure (5) tournées du côté de la partie de prélèvement d'échantillon (3), la partie de capteur (2) comporte des moyens d'accouplement mécanique (11, 56) pour raccorder directement la partie de prélèvement d'échantillon (3) à la partie de capteur (2), les moyens d'accouplement (11, 56) d'entrée et de sortie pouvant être fermés par des prolongements coniques (60) de la partie de prélèvement d'échantillon (3), cette partie de prélèvement d'échantillon (3) comportant un canal d'échantillon (17) muni d'un moyen de raccordement (63) pour une canule ainsi que la chambre de réception (43) et après enlèvement de la partie de prélèvement d'échantillon (3) par rapport à la partie de capteur (2), sa rotation de 180° et sa mise en place sur la partie de capteur, des moyens de raccordement (61, 62) en saillie de la chambre de réception (43) et du canal d'échantillon (17) viennent prendre dans les moyens d'accouplement (11, 56) de la partie de capteur (2) pour qu'après raccordement, l'échantillon coule du canal d'échantillon (17) dans le canal de mesure (5).

20. Elément de mesure jetable selon l'une des revendications 1 à 19, caractérisé en ce que le canal de mesure (5) de la partie de capteur (2) comporte une zone (45, 46) prévue en aval de la plage de mesures (5) suivant le sens d'écoulement de l'échantillon, pour mesurer la température et le niveau de remplissage.

21. Elément de mesure jetable selon l'une des revendications 1 à 20, caractérisé par un capteur optique (7) prévu dans la zone de mesure (6) du canal de mesure (5).

22. Elément de mesure jetable selon la revendication 21, caractérisé par des capteurs optiques (7) placés dans le canal de mesure (5) de la partie de capteur (2) pour l'analyse d'échantillons sanguins, pour mesurer la valer du pH, la teneur en CO₂ et la teneur en O₂.
